# EUROPÄISCHE PATENTSCHRIFT

(11) **EP 1 069 078 B1**
(45) Veröffentlichungstag und Bekanntmachung des Hinweises auf die Patenterteilung: **20.10.2004**
(21) Anmeldenummer: 00114746.1
(22) Anmeldetag: 08.07.2000
(51) Int. Cl.: C02F 1/42, B01J 49/00, C07C 51/47

(54) **Verfahren zur Eluierung fluorierter Emulgatoren**
Method of elution of fluorinated emulgators
Procédé d'elution d'émulgateurs fluorés

(30) Priorität: 14.07.1999 DE 19932771
(43) Veröffentlichungstag der Anmeldung: 17.01.2001
(73) Patentinhaber: Dyneon GmbH & Co. KG, 84504 Burgkirchen (DE)
(72) Erfinder: Führer, Stephan, 84556 Kastl (DE); Löhr, Gernot, Dr., 84508 Burgkirchen (DE); Schwertfeger, Werner, Dr., 84503 Altötting (DE)
(74) Vertreter: Voortmans, Gilbert J.L., Dr.

(56) Entgegenhaltungen:
- EP-A- 0 014 431
- US-A- 3 882 153

## Beschreibung

Die Erfindung betrifft ein Verfahren zur Eluierung fluorierter Emulgatoren von Anionen-Austauscherharzen, entsprechende Mischungen zur Ausführung dieses Verfahrens sowie die weitere Aufarbeitung des Eluats.

Insbesondere betrifft die Erfindung ein Verfahren zur Eluierung fluorierter Emulgatoren, die an ein Anionen-Austauscherharz gebunden sind, wobei das Harz mit einer Mischung in Kontakt gebracht wird, die im wesentlichen
a) Wasser,
b) eine Verbindung der Formel

   M-X

   in welcher M ein Alkalimetall oder Alkylammoniumion ist und X Hydroxyl, Fluorid oder Chlorid bedeuten, und
c) mindestens ein organisches Lösemittel enthält, das die anderen Komponenten a) und b) vollständig löst und so hinreichend viele Anionen X⁻ zur Eluierung der Emulgatoren von dem Anionen-Austauscherharz bereitstellt.

Die Mischung für die Elution besteht vorzugsweise im wesentlichen, in Gewichtsprozent, aus
a) 15 bis 40 % Wasser,
b) 1 bis 10 % der Verbindung M-X und
c) 60 bis 70 % des Lösemittels.

Eine besonders bevorzugte Mischung besteht im wesentlichen aus
a) 18 bis 35 % Wasser,
b) 2 bis 8 % M-X und
c) 60 bis 70 % Lösemittel.

Bevorzugte Lösemittel, die einzeln oder im Gemisch eingesetzt werden können, sind Alkanole mit 1 bis 4 Kohlenstoffatomen, Aceton, Mono- und Dialkylether des Monoglykols und Diglykols, wobei hier unter Alkylgruppen Methyl oder Ethyl verstanden wird. Besonders bevorzugte Lösemittel sind Methanol, Dimethylmonoglykolether und Dimethyldiglykolether.

Bevorzugte Kationen M⁺ sind Lithium, Natrium, Kalium, Tetramethyl- oder Tetraethylammonium und das bevorzugte Anion X⁻ ist Hydroxyl. Überraschenderweise sind besonders effektiv wäßrige Lösungen von Alkalihydroxiden und Ammoniak.

Die zu eluierenden fluorierten Emulgatoren sind seit langem bekannt und werden vor allem in der Polymerisation fluorierter Olefine eingesetzt, da sie nicht telogen wirken. Es handelt sich im wesentlichen um fluorierte Alkancarbonund -sulfonsäuren, wobei der Alkylrest teilweise oder vorzugsweise vollständig fluoriert ist und im allgemeinen linear oder endständig verzweigt ist. Besonders bevorzugt ist die Perfluoroctansäure, im folgenden PFOS genannt, wobei hier im folgenden unter dieser Abkürzung auch die anderen üblichen fluorierten Emulgatoren verstanden werden sollen.

Die Rückgewinnung von PFOS mit Anionen-Austauscherharzen ist seit langem bekannt und beispielsweise in der US-A-3 882 153 und in der EP-B-0 014 431 beschrieben. Besonders vorteilhafte Rückgewinnungsverfahren von PFOS aus Abwasser werden in WO-A-99/62858 und WO-A-99/62830 beschrieben.

Anionen-Austauscherharze sind sehr effektiv, um PFOS aus wäßrigen Systemen zu entfernen. Insbesondere stark basische Anionen-Austauscherharze entfernen praktisch quantitativ PFOS aus Abwasser und ähnlichen Lösungen. In der Praxis können mehr als 95 % der vorhandenen PFOS so zurückgewonnen werden. Es kann die gesamte Kapazität des Ionenaustauschers ausgenutzt werden.

Die Stärke der Adsorption der PFOS an das Austauscherharz erschwert jedoch die Elution. Eluiert man beispielsweise einen beladenen, stark basischen Anionenaustauscher mit einer einmolaren, wäßrigen Lösung von Ammoniak, Natriumhydroxid oder Kaliumfluorid, so erhält man PFOS-Konzentrationen im Eluat in der Größenordnung von nur 0,1 mmol/l. Die Aufarbeitung so erheblicher Volumen von Abwasser ist jedoch nicht wirtschaftlich.

Andererseits sind schwachbasische Anionen-Austauscherharze nicht so effizient in der Rückgewinnung der PFOS aus dem wäßrigen System. So zeigen diese Harze einen frühzeitigen "diffusen" Durchbruch, das heißt die PFOS wird wieder in geringen Mengen in das behandelte wäßrige System abgegeben. Es ist so nicht möglich, die PFOS sicher aus großen Volumina Abwasser unter etwa 5 ppm (entsprechend 7,5 · 10⁻⁶ mol/l) zu reduzieren. Solch niedrige Konzentrationen sind aus Umweltschutzgründen erwünscht, da PFOS biologisch schwer abbaubar ist. Dies trifft insbesondere für Abwässer zu, die nichtionische Emulgatoren enthalten, wie sie beispielsweise bei der Aufkonzentration durch Ultrafiltration eingesetzt werden (EP-B-0 632 009).

Bei dem aus der EP-B-0 014 431 bekannten Verfahren wird die PFOS aus dem Anionen-Austauscher mit einer Mischung aus einem wasserlöslichen Lösemittel wie Methanol und kleinen Mengen einer Mineralsäure wie Schwefelsäure oder Salzsäure eluiert. Man erreicht so mit einer Mischung aus 89 Gew.-% Methanol, 4 Gew.-% Schwefelsäure und 7 Gew.-% Wasser ein Eluat mit bis zu 400 mmol/l PFOS. Üblicherweise trennt sich das Eluat in zwei Schichten, wobei die untere Schicht im wesentlichen aus PFOS besteht und die Oberschicht etwa der Elutionsmischung entspricht. Im größeren Maßstab tritt jedoch diese Phasentrennung nicht verläßlich ein, so daß die Wiedergewinnung der Oberphase zur Elution nicht ohne weiteres gegeben ist (und so beispielsweise ein kontinuierliches Verfahren erschwert wird). Zur Regeneration des Ionenaustauscherharzes werden etwa 5 Bett-Volumen Eluatmischung benötigt, was in der Praxis etwa 5 bis 10 m³ feuergefährliche Eluatmischung bedeutet. Der damit verbundene apparative Aufwand ist beträchtlich.

Erfindungsgemäß wird demgegenüber die vorstehend genannte Mischung eingesetzt, die diese Nachteile nicht aufweist. Darüber hinaus erlaubt das erfindungsgemäße Verfahren eine einfache und effektive Aufarbeitung des Eluats, wobei dieses zunächst einer Wasserdampfdestillation unterzogen wird, bis es im wesentlichen frei von flüchtigen Substanzen ist, worauf im Dampfdestillationsrückstand mit einer hinreichend starken Säure die Emulgatorsäure freigesetzt wird. Vorteilhaft wird dann die freigesetzte Emulgatorsäure abdestilliert und zweckmäßig in wäßriger Ammoniaklösung aufgefangen, da die Emulgatorsäure üblicherweise in Form des Ammoniumsalzes bei der Polymerisation fluorierter Olefine eingesetzt wird.

Die Erfindung betrifft somit ein wirtschaftliches und im großen Maßstab sicher beherrschbares Verfahren zur Elution fluorierter Emulgatoren und ihrer Rückgewinnung in einer so reinen Form, daß sie die Anforderungen erfüllt, die an den Einsatz bei der Polymerisation gestellt werden.

Die Erfindung wird in den folgenden Beispielen näher erläutert.

### Beispiele

Die Elutionskraft der folgenden Elutionsmischungen wurde durch Messung der Konzentration von PFOS im Eluat gemessen, nachdem das erste Bett-Volumen der Elutionsmischung die Ionenaustauschersäule durchströmt hatte. Die Säule hatte eine Länge von 30 cm und einen Durchmesser von 6,5 cm und war mit 400 ml des handelsüblichen Anionenaustauscherharzes ®AMBERLITE 402 (starkbasisch) gefüllt. Das Harz wurde bis zum Durchbruch mit einer Ammoniumperfluoroctanoat-(APFOA)-Lösung (Handelsprodukt der Firma 3M mit der Handelsbezeichnung FC 143) beladen und mit 2 l deionisiertem Wasser gewaschen.

Nachdem 600 ml der Eluatmischung durch die Kolonne geflossen waren, wurde eine Probe entnommen und der Gehalt an PFOS durch Überführen in den Methylester unter gaschromatographischer Analyse unter Verwendung eines internen Standards analysiert. Hierbei liegt die Nachweisgrenze bei 5 ppm.

In den folgenden Beispielen ist der Gehalt an PFOS in ppm angegeben. Die Elutionsgeschwindigkeit war 200 ml/h.

Die Zusammensetzung der Elutionsmischungen ist in Gewichtsprozent angegeben. Bei allen eingesetzten Elutionsmischungen traten keine Mischungslücken auf. Sofern keine anderen Angaben gemacht sind, erfolgte die Elution bei Zimmertemperatur.

### Vergleichsbeispiel (Raumtemperatur)

Elution von PFOS mit wäßrigen Lösungen.

| | | | |
|---|---|---|---|
| Eluent (Wasser) | 1 mol/l NaOH | 1 mol/l NH₃ | 1 mol/l KF |
| Eluatkonzentration an PFOS in ppm | 20 | 17 | 160 |

### Beispiele 1 bis 3

Elution mit wäßrig-organischen Lösungen.

Eine Elutionskonzentration von APFOA von mehr als 30 000 ppm wird als technisch akzeptabel angesehen.

| Beispiel 1 | | | | | |
|---|---|---|---|---|---|
| Zusammensetzung [Gew.-%] | NaOH | 5,0 | 5,0 | 1,0 | 5,0 |
| | NH₃ | - | - | - | 1,7 |
| | H₂O | 25,0 | 45,0 | 26,1 | 23,3 |
| | CH₃OH | 70,0 | 50,0 | 72,9 | 70,0 |

| Elutionstemperatur | Eluatkonzentration an PFOS in ppm | | | | |
|---|---|---|---|---|---|
| Raumtemperatur | | 15 000 | 2 900 | 8 400 | 62 000 |
| 40 °C | | 54 000 | 7 300 | 13 000 | 79 000 |
| 50 °C | | 52 000 | - | - | - |

| Beispiel 2 | | | | | |
|---|---|---|---|---|---|
| Zusammensetzung [Gew.-%] | NH₃ | 2,5 | [(CH₃)₄N]OH | 3,1 | 6,2 |
| | H₂O | 7,5 | H₂O | 31,6 | 24,8 |
| | CH₃OH | 90,0 | CH₃OH | 65,3 | 70,0 |

| Elutionstemperatur | Eluatkonzentration an PFOS in ppm | | | | |
|---|---|---|---|---|---|
| Raumtemperatur | | 2 100 | | 67 000 | 150 000 |
| 40 °C | | 2 800 | | 57 000 | - |
| 50 °C | | 3 100 | | - | - |

| Beispiel 3 | | | | | | |
|---|---|---|---|---|---|---|
| Zusammensetzung [Gew.-%] | NaOH | 4,6 | 4,1 | 4,6 | 3,7 | 4,6 |
| | H₂O | 22,7 | 19,4 | 22,7 | 26,7 | 22,7 |
| | CH₃OH | 63,6 | 35,3 | 63,6 | 32,2 | 63,6 |
| | OS2 *) | 9,1 ME | 41,1 ME | 9,1 DE | 37,4 DE | 9,1 Ac |
| Eluatkonzentration in ppm | PFOS | 170 000 | 140 000 | 33 000 | 57 000 | 26 000 |

| | | | | | | |
|---|---|---|---|---|---|---|
| *) OS2 = Zweites organisches Lösemittel ME = Dimethylmonoglykolether DE = Dimethyldiglykolether Ac = Aceton | | | | | | |

## Patentansprüche

1. Verfahren zum Eluieren eines an ein anionisches Austauscherharz gebundenen fluorierten Emulgators, **dadurch gekennzeichnet, daß** man das Austauscherharz mit einer Mischung in Kontakt bringt, die
a) Wasser,
b) eine Verbindung der Formel
M-X
in welcher M ein Alkalimetall oder ein Alkylammoniumion mit Alkylresten von 1 bis 4 Kohlenstoffatomen und X Hydroxyl, Fluorid oder Chlorid bedeuten, und
c) mindestens ein organisches Lösungsmittel enthält, das die anderen Komponenten a) und b) vollständig löst und so genügend Anionen X⁻ zur Elution des Emulgators von Anionen-Austauscherharz bereitstellt.

2. Verfahren nach Anspruch 1, worin die Mischung im wesentlichen, in Gewichtsprozent, aus
a) 15 bis 40 % Wasser,
b) 1 bis 10 % der Verbindung M-X und
c) 60 bis 70 % Lösemittel
enthält.

3. Verfahren nach Anspruch 2, worin die Zusammensetzung wie folgt ist:
a) 18 bis 35 %,
b) 2 bis 8 % und
c) 60 bis 70 %.

4. Verfahren nach einem oder mehreren der vorhergehenden Ansprüche, in denen das Lösemittel aus der Gruppe der Alkanole mit 1 bis 4 Kohlenstoffatomen, Aceton, Mono- und Dialkylether des Monoglykols und Diglykols ausgewählt ist, wobei der Alkyletherrest hier Methyl oder Ethyl ist.

5. Verfahren nach Anspruch 4, wobei das Lösemittel Methanol und/oder Dimethylmonoglykolether oder Dimethyldiglykolether ist.

6. Verfahren nach einem oder mehreren der vorhergehenden Ansprüche, wobei in der Verbindung M-X M Lithium, Natrium, Kalium, Tetramethyl- oder Tetraethylammonium und X Hydroxid sind.

7. Verfahren nach einem oder mehreren der vorhergehenden Ansprüche, **dadurch gekennzeichnet, daß** als Komponente b) eine ammoniakalische Alkalilauge eingesetzt wird.

8. Verfahren zur Aufarbeitung des nach Anspruch 1 erhaltenen Eluats, **dadurch gekennzeichnet, daß**
a) das Eluat dampfdestilliert wird, bis es im wesentlichen frei von flüchtigen Bestandteilen ist und
b) im Dampfdestillationsrückstand den Emulgator als freie Säure mit einer hinreichend starken Säure freisetzt.

9. Verfahren nach Anspruch 8, **dadurch gekennzeichnet, daß** der Emulgator in seiner freien Säureform abdestilliert und in wäßriger Ammoniaklösung als Ammoniumsalz wiedergewonnen wird.

## Claims

1. Process for the elution of a fluorinated emulsifier bound to an anion exchanger resin, **characterized in that** the resin is brought into contact with a mixture comprising
a) water,
b) a compound of the formula
M-X
in which M is an alkali metal or alkylammonium ion containing radicals having 1 to 4 carbon atoms, and X is hydroxyl, fluoride or chloride, and
c) at least one organic solvent which completely dissolves the other components a) and b) and thus provides a sufficient quantity of anions X- for the elution of the emulsifier from the anion exchanger resin.

2. Process according to Claim 1, in which the mixture essentially consists, in per cent by weight, of
a) from 15 to 40% of water,
b) from 1 to 10% of the compound M-X, and
c) from 60 to 70% of solvent.

3. Process according to Claim 2, in which the composition is as follows:
a) from 18 to 35%,
b) from 2 to 8%, and
c) from 60 to 70%.

4. Process according to one or more of the preceding claims, in which the solvent is selected from the group consisting of alkanols having 1 to 4 carbon atoms, acetone, mono- and dialkyl ethers of monoglycol and diglycol, where the alkyl ether radical here is methyl or ethyl.

5. Process according to Claim 4, where the solvent is methanol and/or dimethyl monoglycol ether or dimethyl diglycol ether.

6. Process according to one or more of the preceding claims, where M in the compound M-X is lithium, sodium, potassium, tetramethylammonium or tetraethylammonium, and X is hydroxide.

7. Process according to one or more of the preceding claims, **characterized in that** component b) is an ammoniacal alkali metal hydroxide solution.

8. Process for the work-up of the eluate obtained according to Claim 1, **characterized in that**
a) the eluate is subjected to steam distillation until it is essentially free from volatile constituents, and
b) the emulsifier in the steam distillation residue is liberated as the free acid using a sufficiently strong acid.

9. Process according to Claim 8, **characterized in that** the emulsifier is distilled off in its free acid form and recovered as the ammonium salt in aqueous ammonia solution.

## Revendications

1. Procédé pour l'élution d'émulsifiants fluorés qui sont liés à une résine échangeuse d'anions, **caractérisé en ce qu'**on met la résine échangeuse en contact avec un mélange qui contient
a) de l'eau,
b) un composé de formule
M-X
dans laquelle M représente un métal alcalin ou un ion alkylammonium avec des radicaux alkyle comprenant 1 à 4 atomes de carbone et X signifie hydroxyle, fluorure ou chlorure et
c) au moins un solvant organique qui dissout complètement les autres composants a) et b) et qui met ainsi à disposition suffisamment d'anions X- pour l'élution de l'émulsifiant de la résine échangeuse d'anions.

2. Procédé selon la revendication 1, dans lequel le mélange contient essentiellement, en % en poids,
a) 15 à 40% d'eau,
b) 1 à 10% du composé M-X et
c) 60 à 70% du solvant.

3. Procédé selon la revendication 2, dans lequel la composition est comme suit :
a) 18 à 35%
b) 2 à 8% et
c) 60 à 70%.

4. Procédé selon l'une ou plusieurs des revendications précédentes, dans lequel le solvant est choisi dans le groupe constitué par les alcanols comprenant 1 à 4 atomes de carbone, l'acétone, les monoalkyléthers et les dialkyléthers du monoglycol et du diglycol, le radical alkyléther étant ici méthyle et éthyle.

5. Procédé selon la revendication 4, où le solvant est le méthanol et/ou le diméthylmonoglycoléther ou le diméthyldiglycoléther.

6. Procédé selon l'une ou plusieurs des revendications précédentes, où, dans le composé M-X, M représente lithium, sodium, potassium, tétraméthylammonium ou tétraéthylammonium et X représente hydroxyde.

7. Procédé selon l'une ou plusieurs des revendications précédentes, **caractérisé en ce qu'**on utilise comme composant b) une lessive alcaline ammoniacale.

8. Procédé pour le traitement de l'éluat obtenu selon la revendication 1, **caractérisé en ce que**
a) l'éluat est soumis à une distillation à la vapeur d'eau jusqu'à ce qu'il soit essentiellement exempt des constituants volatils et
b) l'émulsifiant est libéré du résidu de la distillation à la vapeur sous forme d'acide libre avec un acide suffisamment fort.

9. Procédé selon la revendication 8, **caractérisé en ce que** l'émulsifiant est éliminé par distillation sous sa forme acide libre et est récupéré dans une solution aqueuse d'ammoniaque sous forme de sel d'ammonium.
